(19) Europäisches Patentamt
European Patent Office
Office européen des brevets

(11) Publication number: **0 070 171**
**B1**

(12) **EUROPEAN PATENT SPECIFICATION**

(45) Date of publication of patent specification: **15.10.86**

(21) Application number: **82303623.1**

(22) Date of filing: **09.07.82**

(51) Int. Cl.$^4$: **C 07 D 471/10,**
C 07 D 498/10,
A 61 K 31/505, A 61 K 31/535

(54) **Novel piperidine derivatives, processes for preparation thereof and pharmaceutical compositions containing same.**

(30) Priority: **11.07.81 JP 108668/81**

(43) Date of publication of application:
**19.01.83 Bulletin 83/03**

(45) Publication of the grant of the patent:
**15.10.86 Bulletin 86/42**

(84) Designated Contracting States:
**DE FR GB IT**

(56) References cited:
**EP-A-0 018 417**
**EP-A-0 029 707**
**EP-A-0 065 864**
**GB-A-1 343 668**
**US-A-4 092 414**
**US-A-4 209 625**

(73) Proprietor: **KYOWA HAKKO KOGYO CO., LTD**
**Ohtemachi Bldg. Ohtemachi 1-chome**
**Chiyoda-ku**
**Tokyo (JP)**

(72) Inventor: **Teranishi, Masayuki**
**2-13-24, Narusedai**
**Machida-shi Tokyo (JP)**
Inventor: **Obase, Hiroyuki**
**3-9-9, Naka-machi**
**Machida-shi Tokyo (JP)**
Inventor: **Takai, Haruki**
**Green Haitsu Yurigaoka 401 210-13, Takaishi**
**Tama-ku Kawasaki-shi Kanagawa-ken (JP)**
Inventor: **Shuto, Katsuichi**
**410-1, Nameri, Nagaizumi-cho**
**Sunto-gun Shiquoka-ken (JP)**
Inventor: **Karasawa, Akira**
**411-3, Shimonagakubo Nagaizumi-cho**
**Sunto-gun Shizuoka-ken (JP)**
Inventor: **Kasuya, Yutaka**
**1-61, Komukainishi-machi Saiwai-ku**
**Kawasaki-shi Kanagawa-ken (JP)**

(74) Representative: **Lambert, Hugh Richmond et al**
**D. YOUNG & CO. 10 Staple Inn**
**London, WC1V 7RD (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 070 171 B1

**Description**

The present invention relates to novel piperidine derivatives, acid addition salts thereof and processes for preparation thereof. The above compounds have a hypotensive activity.

The present invention also relates to pharmaceutical compositions containing the same.

Heretofore, many piperidine derivatives having a hypotensive activity are disclosed in patent applications. Namely, European Patent Publication No. 18417 published on November 12, 1980 discloses the compounds represented by the following formula:

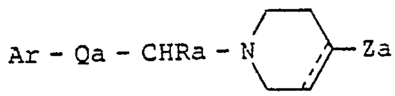

[wherein Ar is phenyl, substituted phenyl, etc.; Qa is carbonyl, methylene, hydroxymethylene, etc.; Ra is hydrogen or alkyl; and Za is

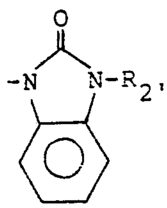

(wherein $R_{2'}$ is hydrogen, etc.)

or

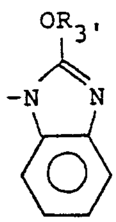

(wherein $R_{3'}$ is alkyl)]

Further, European Patent Publication No. 29707 published on June 3, 1981 discloses the compounds represented by the following formula:

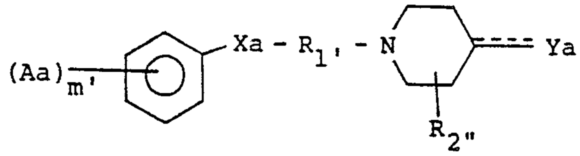

[wherein Aa is hydroxyl, halogen; lower alkyl, lower alkoxy, etc. or two Aa groups may combine to form lower alkylenedioxy; m' is 0 or an integer of 1—5; Xa is oxygen, sulfur, carbonyl, hydroxymethylene or methylene; $R_{1'}$ is straight-chain alkylene having 1—4 carbon atoms with or without lower alkyl substituent(s); $R_{2''}$ is hydrogen or lower alkyl; and Ya is

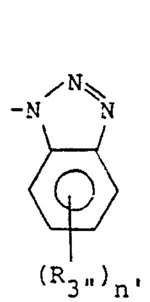 , 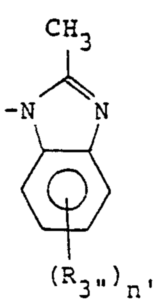 , 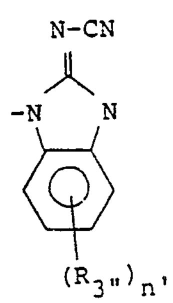 , 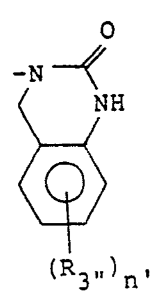 ,

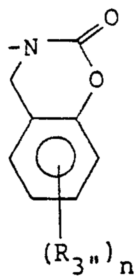 , 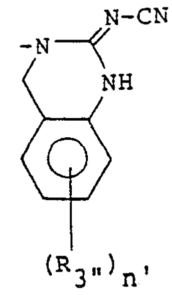 , 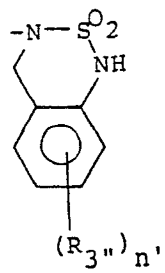 , 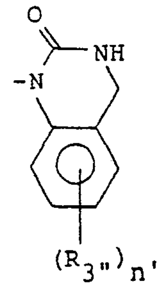 ,

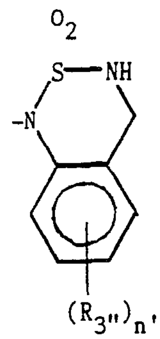 , 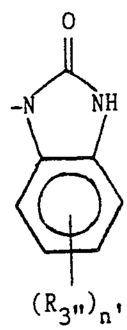 or 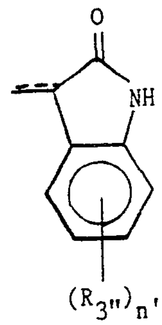

(wherein $R_{3''}$ is hydroxy, lower alkoxy, halogen, etc.)].

And still further, European Patent Publication No. 65864 published on December 1, 1982 discloses compounds represented by the formula:

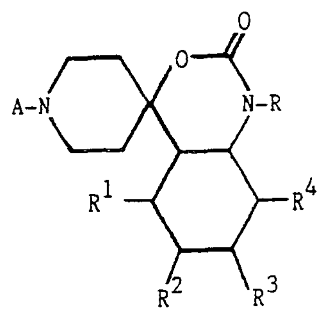

wherein

$R^1$, $R^2$, $R^3$, and $R^4$ are each independently hydrogen, hydroxy, lower alkyl, lower alkoxy or halo;

R is hydrogen or lower alkyl; and

A is selected from:

2-(benzodioxan-2-yl)-2-hydroxyethyl;

ω-(benzodioxan-2-yl)-alkyl (where alkyl is a straight or branched chain hydrocarbon of 1—4 carbons);

3-(aryloxy)-2-hydroxypropyl, wherein aryloxy is phenyloxy optionally substituted by 1, 2 or 3 moieties selected from lower alkyl, lower alkoxy, halo, lower alkylsulfamido, lower alkkoxycarbonyl, cyano and trifluoromethyl;

ω-arylalkyl wherein alkyl is as above defined and wherein aryl is phenyl optionally substituted by 1, 2 or 3 moieties selected from lower alkyl, lower alkoxy, halo, lower alkylsulfamido, lower alkoxycarbonyl, cyano and trifluoromethyl;

ω-aryl-ω-oxoalkyl, wherein aryl and alkyl are as above defined;

ω-aryl-ω-hydroxyalkyl, wherein alkyl and aryl are as above defined; and

ω-aryloxyalkyl, wherein alkyl and aryloxy are as above defined.

The present invention relates to still other piperidine derivatives, similar in structure to those disclosed in European Publication No. 65864, and having hypotensive activity, or useful as intermediates in the manufacture of other compounds falling within the general formula and which have hypotensive activity although the intermediates themselves do not.

Broadly speaking the novel piperidine derivatives of this invention are compounds of the formula [I]:

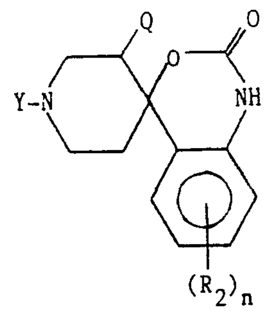

[I]

wherein Y is $C_1$—$C_3$ alkyl or a group represented by

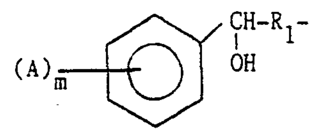

wherein m is 0 or an integer of 1—5; A is hydroxyl, halogen, $C_1$—$C_5$ alkyl, $C_1$—$C_5$ alkoxy, $C_2$—$C_5$ alkenyloxy, $C_2$—$C_5$ alkynyloxy, $C_1$—$C_5$ alkylthio, carboxyl, $C_1$—$C_5$ alkoxycarbonyl, nitro, amino, $C_1$—$C_5$ alkylamino, $C_1$—$C_5$alkanoylamino, sulfamoyl, mono or di-($C_1$—$C_5$ alkyl) amino-sulfonyl, $C_1$—$C_5$ alkylsulfonyl, carbamoyl, cyano or trifluoromethyl; when m is 2 or more, the A groups may be the same or different, or two A groups may be combined to form a $C_1$—$C_5$ alkylenedioxy group; $R_1$ is straight-chain alkylene having 1—4 carbon atoms, or straight-chain alkylene having 1—4 carbon atoms and having a $C_1$—$C_5$ alkyl substituent; Q is hydrogen, $C_1$—$C_5$ alkyl, halogen, hydroxyl or $C_1$—$C_5$ alkanoyloxy; n is 0 or an integer of 1—4; $R_2$ is hydroxyl, $C_1$—$C_5$ alkoxy, halogen, trifluoromethyl, trifluoromethoxy, trifluoromethylthio, nitro or amino; when n is 2 or more, the $R_2$ groups may be the same or different, or two $R_2$ groups may be combined to form a $C_1$—$C_5$ alkylenedioxy.

However, in view of European Publication No. 65864, certain provisos apply, namely that when Y is said

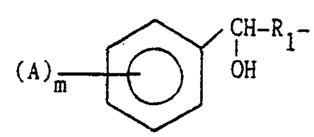

group,

then Q is H and, if none of the $R_2$ groups is halogen, at least two of the A groups in

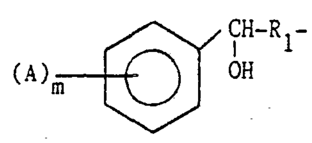

must be alkoxy.

The term "halogen" is the definition of A, Q, R and $R_2$ in Compound [I] includes chlorine and bromine, and where $C_1$—$C_5$ alkyl occurs in any definition, the preferred alkyl groups are those containing from 1—3 carbon atoms.

Compound [I] includes all the optical isomers.

In addition to the foregoing, the present invention also includes the acid addition salts of the Compounds I. Examples of acid addition salts of Compound [I] are various inorganic acid addition salts such as hydrochloride, hydrobromide, hydroiodide, nitrate, sulfate and phosphate, and varous organic acid addition salts such as formate, acetate, benzoate, maleate, fumarate, succinate, tartrate, citrate, oxalate, glyoxylate, aspartate, methanesulfonate, ethanesulfonate, propanesulfonate, methanesulfonate, α,β-ethanedisulfonate and benzenesulfonate.

More specifically, the present invention provides a piperidine derivative represented by the general formula [I']:

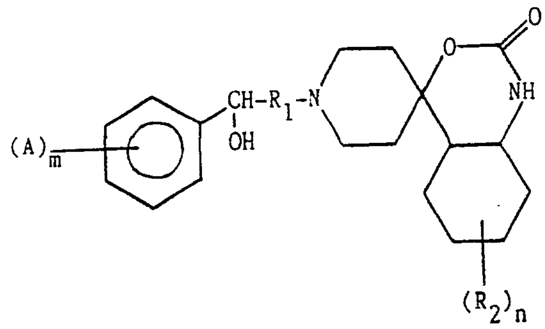

[I']

wherein A, $R_1$ $R_2$, m and n have the same meanings as defined above, but subject to the same provisos, and pharmacologically acceptable acid addition salts thereof, and a piperidine derivative represented by the general formula (I''')

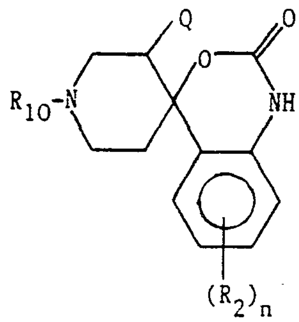

[I''']

(wherein $R_{10}$ is $C_1$—$C_3$ alkyl, and Q, $R_2$ and n have the same meanings as defined above) and acid addition salts thereof.

Compounds [I'] and pharmacologically acceptable acid addition salts thereof have pharmacological acitivities, especially a hypotensive activity and are expected to be useful as a medicament. Examples of pharmacologically acceptable acid addition salts of Compound [I'] are the same as those of acid addition salts of Compound [I].

Compound [I'''] is useful as an intermediate for synthesis of Compound [I'].

Examples of acid addition salts of Compound [I'''] are the same as those of Compound [I].

Especially preferred compounds within the formula representing Compound [I'] are represented by the general formula [I'']:

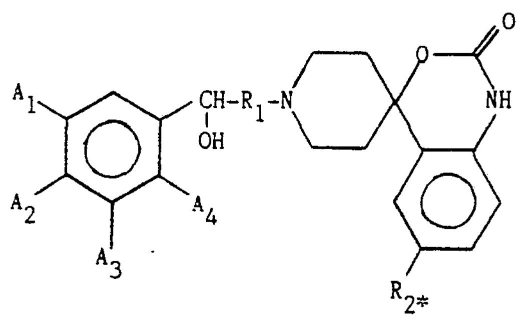

[I'']

wherein $A_1$, $A_2$, $A_3$ and $A_4$ are the same or different groups and are hydrogen or a group as defined for A; $R_{2*}$ is hydrogen or a group as defined for $R_2$; and $R_1$ is as defined above, it being provided that either $R_{2*}$ is halogen or at least two of $A_1$—$A_4$ are $C_1$—$C_5$ alkoxy, and their pharmaceutically acceptable acid addition salts.

Typical examples of the compounds of the present invention are shown in Table 1. In Tables 2 and 3, values of $C^{13}$-NMR (found) of typical compounds and their assignments are shown. Physical properties of these compounds are shown in Tables 4 and 5.

TABLE 1—1

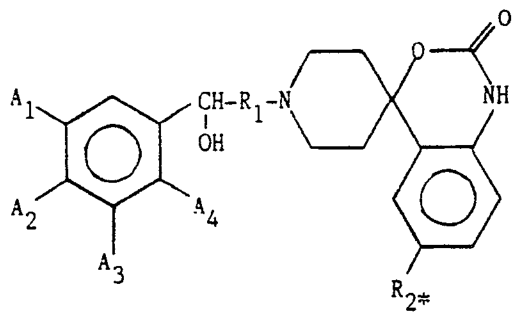

[I'']

| Compound No. Example No. | Compound | | | | | | Form |
|---|---|---|---|---|---|---|---|
| | $A_1$ | $A_2$ | $A_3$ | $A_4$ | $R_1$ | $R_{2*}$ | |
| 4 | $OCH_3$ | $OCH_3$ | H | H | $—CH_2—$ | H | Free |
| 6 | H | H | H | H | $—CH_2—$ | Cl | Free |
| 8 | H | Cl | H | H | $—CH_2—$ | Cl | Free |

TABLE 1—2

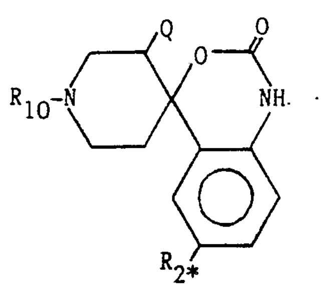

[I''']

| Compound No. (Example No.) | Compound | | | |
|---|---|---|---|---|
| | | Structure | | |
| | Name | $R_{10}$ | $R_{2*}$ | Q |
| 14 | 1'-methyl-(6-chloro-2-oxo-1,2-dihydro-4H-3,1-benzoxazine-4-spiro-4'-piperidine) | $CH_3$ | Cl | H |

TABLE 1—3

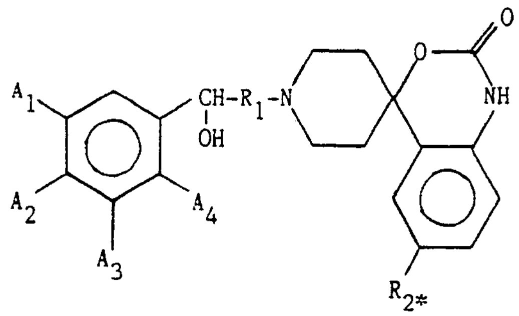

| Compound No. (Example No.) | Compound | | | | Form |
|---|---|---|---|---|---|
| | $A_1$ $A_2$ $A_3$ $A_4$ | $R_1$ | $R_{2*}$ | Configuration | |
| 16 | $OCH_3$ $OCH_3$ H H | $-CH-$<br>$CH_3$ | H | threo | Free |
| 17 | $OCH_3$ $OCH_3$ H H | $-CH-$<br>$CH_3$ | H | erythro | Free |
| 19 | $OCH_3$ $OCH_3$ $OCH_3$ H | $-CH-$<br>$CH_3$ | H | threo | Free |
| 21 | $OCH_3$ $OCH_3$ H $OCH_3$ | $-CH-$<br>$CH_3$ | H | threo | Free |
| 22 | $OCH_3$ $OCH_3$ H $OCH_3$ | $-CH_3-$<br>$CH_3$ | H | erythro | Free |
| 23 | $OCH_3$ $OCH_3$ $OCH_3$ H | $-CH_2-$ | H | | Free |

Names of the compounds with compound numbers in Tables 1—1 and 1—3 are as follows (represented as free compounds):

Compound No. 4: 1′-[2-(3,4-dimethoxyphenyl)-2-hydroxyethyl]-(2-oxo-1,2-dihydro-4H-3,1-benzoxazine-4-spiro-4′-piperidine)

Compound No. 6: 1′-(2-phenyl-2-hydroxyethyl)-(6-chloro-2-oxo-1,2-dihydro-4H-3,1-benzoxazine-4-spiro-4′-piperidine)

Compound No. 8: 1′[2-(4-chlorophenyl)-2-hydroxyethyl]-(6-chloro-2-oxo-1,2-dihydro-4H-3,1-benzoxazine-4-spiro-4′-piperidine)

Compound No. 16: threo-1′-2-(3,4-dimethoxyphenyl)-2-hydroxy-1-methylethyl]-(2-oxo-1,2-dihydro-4H-3,1-benzoxazine-4-spiro-4′-piperidine)

Compound No. 17: erythro-1′-[2-(3,4-dimethoxyphenyl)-2-hydroxy-1-methylethyl]-(2-oxo-1,2-dihydro-4H-3,1-benzoxazine-4-spiro-4′-piperidine)

Compound No. 19: threo-1′-2-(3,4,5-trimethoxyphyenyl)-2-hydroxy-1-methylethyl]-(2-oxo-1,2-dihydro-4H-3,1-benzoxazine-4-spiro-4′-piperidine)

Compound No. 21: threo-1′[2-(3,4,6-trimethoxyphenyl)-2-hydroxy-1-methylethyl]-(2-oxo-1,2-dihydro-4H-3,1-benzoxazine-4-spiro-4′-piperidine)

Compound No. 22: erythro-1′-[2-(3,4,6-trimethoxyphenyl)-2-hydroxy-1-methylethyl]-(2-oxo-1,2-dihydro-4H-3,1-benzoxazine-4-spiro-4′-piperidine)

Compound No. 23: 1′-2-(3,4,5-trimethoxyphenyl)-2-hydroxyethyl]-(2-oxo-1,2-dihydro-4H-3,1-benzoxazine-4-spiro-4′-piperidine)

TABLE 2

Compounds whose $C^{13}$—NMR are measured, position number and solvents used for the measurement

Compound No. 4

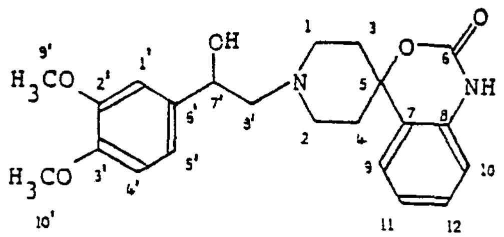

$d_6$-DMSO

Compound No. 8

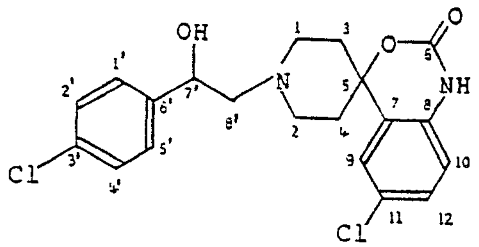

$d_6$-DMSO

TABLE 3

$C^{13}$—NMR (ppm)

| Compound No. \ Position | 1, 2 | 3, 4 | 5 | 6 | 13 | 7′ | 8′ | 9′, 10′ | Aromatic ring 7, 8, 9, 10, 11, 12, 1′, 2′, 3′, 4′, 5′, 6′, |
|---|---|---|---|---|---|---|---|---|---|
| 4 | 47,906<br>49.125 | 34.992 | 80.377 | 150.983 | — | 69.959 | 66.548 | 55.948<br>55.765 | 110.167, 111.872<br>114.614, 118.452<br>123.630, 123.325<br>125.701, 129.174<br>135.326, 137.276<br>148.059, 148.790 |
| 8 | 47.687<br>48.589 | 34.480 | 79.853 | 150.033 | — | 69.277 | 65.866 | | 115.991, 123.496<br>126.737, 127.419<br>127.980, 128.808<br>131.343, 134.291<br>143.575 |

TABLE 4—1

| Compound No. | Chemical Formula | Elemental analysis (%) | | | | | |
|---|---|---|---|---|---|---|---|
| | | Calculated | | | Found | | |
| | | C | H | N | C | H | N |
| 4 | $C_{22}H_{26}N_2O_5$ | 66.32 | 6.58 | 7.03 | 66.44 | 6.61 | 7.05 |
| 6 | $C_{20}H_{21}ClN_2O_3$ | 64.43 | 5.68 | 7.51 | 64.16 | 5.59 | 7.58 |
| 8 | $C_{20}H_{20}Cl_2N_2O_3$ | 58.98 | 4.95 | 6.88 | 58.97 | 4.98 | 7.08 |

TABLE 4—2

| Compound No. | Chemical Formula | Elemental analysis (%) | | | | | |
|---|---|---|---|---|---|---|---|
| | | Calculated | | | Found | | |
| | | C | H | N | C | H | N |
| 14 | $C_{13}H_{15}N_2O_2Cl \cdot HCl$ | 51.50 | 5.32 | 9.24 | 51.28 | 5.41 | 9.06 |

TABLE 4—3

| Compound No. | Chemical Formula | Elemental analysis (%) | | | | | |
|---|---|---|---|---|---|---|---|
| | | Calculated | | | Found | | |
| | | C | H | N | C | H | N |
| 16 | $C_{23}H_{28}N_2O_5$ | 66.97 | 6.84 | 6.79 | 66.71 | 6.95 | 6.84 |
| 17 | $C_{23}H_{28}N_2O_5$ | 66.97 | 6.84 | 6.79 | 67.07 | 6.97 | 6.85 |
| 19 | $C_{24}H_{30}N_2O_6$ | 65.14 | 6.83 | 6.33 | 64.96 | 6.93 | 6.45 |
| 21 | $C_{24}H_{30}N_2O_6$ | 65.14 | 6.83 | 6.33 | 65.39 | 6.99 | 6.43 |
| 22 | $C_{24}H_{30}N_2O_6$ | 65.14 | 6.83 | 6.33 | 64.92 | 6.96 | 6.31 |
| 23 | $C_{32}H_{28}N_2O_6$ | 64.47 | 6.59 | 6.54 | 64.23 | 6.58 | 6.67 |

TABLE 5—1

| Compound No. | Melting Point | IR(KBr) ($cm^{-1}$) | NMR (60MHz) (δ values of main peaks) |
|---|---|---|---|
| 4 | 212.5~213.5°C | 1715 | 1.7—3.15, 3.75, 3.85, 4.4—5.0, 6.78—7.5, 10.20 ($d_6$-DMSO) |
| 6 | 244.8~247.0°C | 1717 | 1.7—3.1, 4.6—5.1, 6.8—7.6, 10.35 ($d_6$-DMSO) |
| 8 | 238.2~240.0°C | 1710 | 1.7—3.1, 4.6—5.2, 6.83—7.6, 7.4, 10.33 ($d_6$-DMSO) |

TABLE 5—2

| Compound No. | Form | Melting point (°C) | IR(KBr) ($cm^{-1}$) | PMR (ppm) (100 MHz) (δ value of main peaks) |
|---|---|---|---|---|
| 14 | HCl | >300 | 1710 | 2.13, 2.28, 2.85, 6.95, 7.03, 7.23, 7.33, 7.35, 7.41, 7.44, 10.59, 11.26, (broad) ($d_6$-DMSO) |

TABLE 5—3

| Compound No. | Melting Point (°C) | IR(KBr) ($cm^{-1}$) | NMR (90MHz) (δ values of main peaks) |
|---|---|---|---|
| 16 | 151.5—153.2 | 1715 | 0.85 (doublet, J=6Hz), 2.0—2.3, 2.5—2.9, 3.06—3.43, 3.85, 3.86, 4.24 (doublet, J=10Hz), 6.7—7.3, 9.0 ($CDCl_3$) |
| 17 | 200.0—202.8 | 1700 | 0.95 (doublet, J=7Hz), 2.0—2.3, 2.66—3.13, 3.86, 3.88, 4.87 (doublet, J=4.5Hz), 6.76—7.3, 8.9 ($CDCl_3$) |
| 19 | 241.2—243.0 | 1700 | 0.91 (doublet, J=7Hz), 2.0—2.4, 2.5—2.95, 3.02—3.43, 3.83, 3.86, 4.22 (doublet, J=10Hz), 6.60, 6.80—7.36, 9.0 ($CDCl_3$) |
| 21 | 209.6—210.2 | 1700 | 0.85 (doublet, J=7Hz), 2.0—2.35, 2.5—2.96, 3.03—3.45, 3.80, 3.85, 3.88, 4.84 (doublet, J=10Hz), 6.49, 6.76—7.33, 8.90 ($CDCl_3$) |
| 22 | 210.9—212.0 | 1700 | 0.92 (doublet, J=7Hz), 1.95—2.35, 2.73—3.3, 3.79, 3.85, 3.89, 5.22 (doublet, J=3Hz), 6.48, 6.8—7.3, 8.85 ($CDCl_3$) |
| 23 | 224.5—226.0 | 1720 | 2.0—2.3, 2.43—3.2, 3.83, 3.86, 4.56—4.8, 6.60, 6.73—7.36, 8.83 ($CDCl_3$) |

Hypotensive activity of typical compounds [1] is illustrated below as experiment.

Experiment 1

This experiment was conducted according to the procedure described in "Spontaneously Hypertensive Rats (SHR) guidelines for Breeding, Care and Use" (published by SHR Conference) (1976) p.11.

Five spontaneously hypersensitive rats (15 weeks old, 180 mmHg or more in blood pressure) were used as one group. A test compound was suspended on 0.3% (W/V) CMC aqueous solution in a concentration of 3 mg/ml, and orally administered to the rats in a dose of 3 mg/100 g body weight. Changes in blood pressure were measured according to the method of tail artery plethysmography (see the literature cited above). The maximum reduction (mmHg) in blood pressure after the administration on the basis of the pressure before the administration is shown in Table 6.

TABLE 6

| Compound No. | Reduction in blood pressure (mmHG) |
|---|---|
| 4 | 95.0 126.6 millibars |
| 6 | 66.3 88.4 millibars |
| 8 | 67.5 90.0 millibars |
| 16 | 90.0 120.0 millibars |
| 17 | 66.0 88.0 millibars |
| 19 | 70.0 93.3 millibars |
| 21 | 85.0 113.3 millibars |
| 22 | 75.0 100.0 millibars |
| 23 | 79.0 105.3 millibars |

Experiment 2

Three or four male dd-strain mice (weight 18—20 g) were used as one group.

Each of the compounds was added to aqueous physiological sodium chloride, and the mixture was orally administered to the mice in a dose of 300 mg/kg or further 200, 100 or 1000 mg/kg. After the observation for 7 days, the number of deaths were counted. Doses at which half the test animals were dead are shown in Table 7.

TABLE 7

| Compound No. | Dose at which half the mice died (mg/kg) |
|---|---|
| 4 | more than 1000 |
| 8 | more than 300 |

The compounds of the present invention may be prepared as follows:

(1) Compounds of the formula [I']

Compound [I'] can be prepared by reacting a piperidine derivative represented by the general formula [II]:

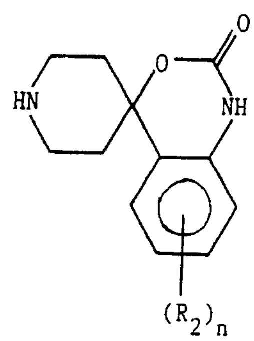

[II]

(wherein n and $R_2$ have the same meaning as defined above except that any hydroxyl or amino groups present as $R_2$ should be suitably protected) with a compound represented by the general formula (III):

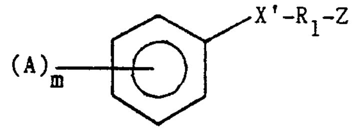

[III]

[wherein A, m and $R_1$ have the same meanings as defined above except that any hydroxyl, amino or lower alkylamino group present as A should be suitably protected, X′ is carbonyl

( 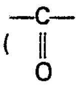 ),

and Z is halogen or an eliminable group] in an inert solvent, reducing the carbonyl group in the initial reaction product, and if necessary, eliminating any protective groups therefrom.

In the definition of Z in compound [III], halogen includes chlorine, bromine or iodine, and the eliminable group includes alkylsulfonyloxy (for example, methanesulfonyloxy), arylsulfonyloxy (for example, benzenesulfonyloxy and paratoluenesulfonyloxy). The inert solvent includes ketone (for example, acetone), halogenated hydrocarbon (for example, chloroform and methylene chloride), amide (for example dimethylformamide), sulfoxide (for example, dimethylsulfoxide), substituted or unsubstituted aromatic hydrocarbon (for example, benzene, toluene and chlorobenzene), lower alcohol (for example, methanol, ethanol and isopropanol), etc. alone or in combination thereof.

The reaction is carried out at 0—150°C, preferably at a temperature between room temperature and the boiling point of the solvent to be used depending on the reactivity of the group Z which is exchangeable.

In general, when the reaction is conducted in the presence of a base, the reaction can be more smoothly carried out. The base to be used includes lower alcoholate (for example, sodium methylate and sodium ethylate), alkali hydroxide (for example, sodium hydroxide), alkali carbonate (for example, sodium carbonate and potassium carbonate), tertiary amine (for example, triethylamine and pyridine). The amount of the base to be used is 1.0 to 1.2 equivalent weights to that of Compound [II] (when an acid addition salt such as hydrochloride of Compound [II] is used, the base must be, of course, excessively added in an amount necessary for neutralizing the acid).

Use of a reaction-promoting agent such as potassium iodide is preferable for smooth proceeding of the reaction.

The reaction is usually completed in 30 minutes to 48 hours. Particularly under reflux with heating, it is completed in 30 minutes to 30 hours.

When any of $R_2$ and A is hydroxyl, amino or lower alkylamino, these substituents are protected according to a conventional procedure before the reaction. After the reaction, the protecting group is eliminated according to a conventional procedure to obtain the desired compound.

In reducing the intermediate product to obtain Compound [I′] the reaction is carried out by reacting the intermediate with a complex metal hydride such as sodium borohydride in a lower alcohol such as methanol, ethanol and isopropanol at −10 to 100°C, preferably at a temperature between 0°C and the boiling point of the used solvent. Alternatively, the reaction can be carried out by subjecting the intermediate to catalytic reduction using a hydrogenating catalyst such as palladium carbon in lower alcohol such as methanol and ethanol, lower aliphatic acid such as acetic acid, water or a mixed solvent thereof. These reactions can be carried out in an open vessel or in a closed vessel under pressure.

Isolation and purification of Compound [I′] are carried out according to ordinary procedures as used in the field of organic synthetic chemistry, for example, concentration, extraction, recrystallization and chromatography. Specifically, since Compound [I′] is generally ready to crystallize, it can be isolated and purified by distilling off the solvent from the reaction mixture and recrystallizing the residue from a suitable solvent such as ethanol.

An acid addition salt of Compound [I′] can be obtained by reaction of compound [I′] with an appropriate acid in an appropriate solvent such as ethanol.

Compound [II], which is a starting compound of Compound [I′], can be obtained according to the following procedure:

First, a piperidine derivative represented by the general formula [IV]:

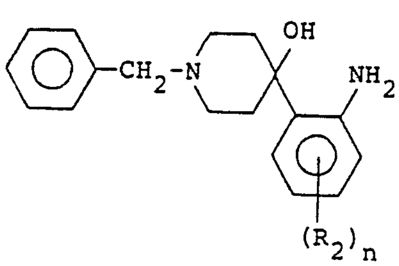

[ IV ]

(wherein $R_2$ and n have the same meanings as defined above) is reacted with a compound selected from a carbonic acid halide such as phosgene, trichloromethyl chloroformate and alkyl chlorocarbonate, a carbonic acid diester such as diphenyl carbonate, 1,1′-carbonyldiimidazole and so on to obtain a compound represented by the general formula [VI]:

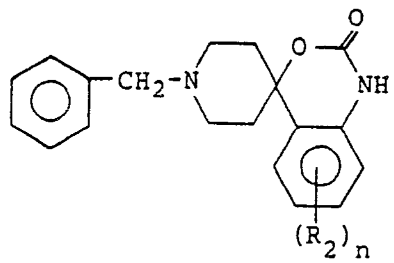

[ VI ]

(wherein $R_2$ and n have the same meanings as defined above).

The above reactions can be carried out according to the known procedure and the reaction using 1,1′-carbonyldiimidazole is described below. The reaction is carried out in an aprotic polar solvent such as halogenated hydrocarbon (for example, methylene chloride and chloroform), ether (for example, ethyl ether, tetrahydrofuran and dioxane), acetonitrile, dimethylformamide and dimethylsulfoxide, alone or in combination thereof preferably with stirring.

The amount of 1,1′-carbonyldiimidazole to be used is preferably 1.0—2.0 equivalent weights to that of Compound [IV]. An appropriate reaction temperature is between room temperature and the boiling point of the solvent.

Reaction time is usually 1—3 hours when the reaction is carried out at the boiling point of the solvent, and 8—12 hours when it is carried out at room temperature. Compound [IV] obtained is then subjected to elimination reaction of the benzyl group to obtain Compound [II—1].

For the elimination of the benzyl group, a procedure using alkyl chlorocarbonate such as vinyl chlorocarbonate, a catalytic reduction, or other ordinary procedures for elimination of N-benzyl group can be used. The catalytic reduction is described below. Compound [VI] is catalytically reduced in the presence of a reducing catalyst such as a palladium catalyst, e.g., palladium carbon in lower alcohol such as methanol, ethanol and isopropanol, water, or a mixed solvent thereof. The reaction is carried out in the coexistence of an acid catalyst (for example, a mineral acid such as hydrochloric acid, hydrobromic acid, hydroiodic acid and perchloric acid, or an aliphatic acid such as acetic acid and propionic acid), an appropriate amount of which is 1—2 equivalent weights. An appropriate reaction temperature is at room temperature to 50°C.

Compound [IV], which is the starting compound for the synthesis of Compound [II], is prepared as follows.

A pivaloylaminobenzene derivative represented by the general formula [VIII]:

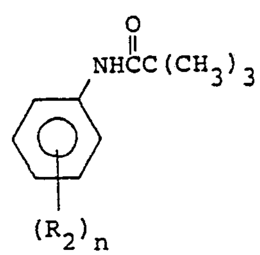

[ VIII ]

(wherein $R_2$ and n have the same meanings as defined above) is converted to a dilithium salt thereof by reaction with a lithium compound-forming agent such as n-butyl lithium and likthium isopropylamide in a mixed solvent of an anhydrous ether (for example, ethyl ether and tetrahydrofuran) and n-hexane, and then the dilithium salt is reacted with a compound represented by the formula [IX]:

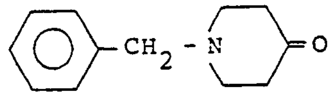

[ IX ]

to form a compound represented by the general formula [X]:

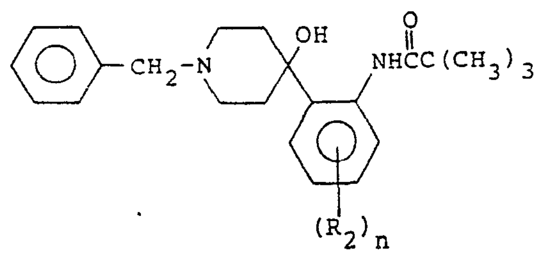

[ X ]

(wherein $R_2$ and n have the same meanings as defined above). In the above reactions, the lithium compound formation is carried out at −5 to 30°C for 2 to 24 hours. The amount of a lithium compound-forming agent is preferably 2—2.5 moles per mole of Compound [VIII]. In the reaction with Compound [IX], an appropriate reaction temperature is −5 to 30°C, and an appropriate reaction time is 2 to 24 hours. The amount of Compound [IX] is preferably 1—1.5 moles per mole of Compound [VIII].

The resulting Compound [X] is hydrolized in a mineral acid (for example, hydrochloric acid, hydrobromic acid, hydroiodic acid and sulfuric acid) to obtain Compound [IV]. The hydrolysis is carried out in the 1 to 10N mineral acid, preferably in the 2 to 6N mineral acid. The appropriate reaction temperature is between 50°C and the boiling point of the reaction solution, and usually at the boiling point of the reaction solution. The reaction is usually completed in 24 to 55 hours.

(2) Compounds of the formula [I''']

Compounds [1'''] may be obtained by reacting a piperidine derivative represented by the general formula [XII]:

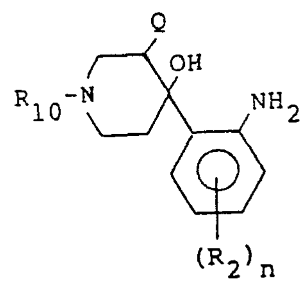

[ XII ]

(wherein $R_{10}$, $R_2$, n and Q have the same meanings as defined above) with a compound selected from a carbonic acid halide (for example, phosgene, trichloromethyl chloroformate and alkyl chlorocarbonate) a carbonic acid diester, 1,1'-carbonyldiimidazole, etc.

The above reaction can be carried out according to the known procedure, and the reaction using 1,1'-carbonyldiimidazole is described below. In this case, the reaction is carried out in an aprotic polar solvent such as halogenated hydrocarbon (for example, methylene chloride and chloroform), ether (for example, ethyl ether, tetrahydrofuran and dioxane), acetonitrile, dimethylformamide and dimethylsulfoxide (alone or in combination thereof) preferably with stirring.

The amount of 1,1-carbonyldiimidazole to be used is 1.0 to 2.0 equivalent weights to that of Compound [II]. An appropriate reaction temperature is room temperature to the boiling point of the solvent.

An appropriate reaction time is 1 to 3 hours when the reaction is carried out at the boiling point of the solvent, and 8 to 12 hours when it is carried out at room temperature.

Compound [XII] which is the starting compound for the synthesis of Compound [I'''], is prepared as follows.

A pivaloylaminobenzene derivative represented by the general formula [XVIII]:

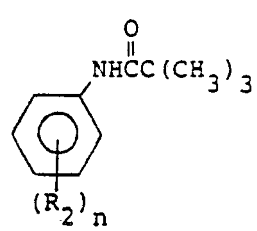

[ XVIII ]

(wherein $R_2$ and n have the same meanings as defined above) is converted to a dilithium salt thereof by the reaction with lithium compound-forming agent wuch as n-butyl lithium and lithium isopropylamide in a mixed solvent of anhydrous ether (ethyl ether or tetrahydrofuran,) and n-hexane. The resulting dilithium salt is reacted with a compound represented by the general formula [XIX]:

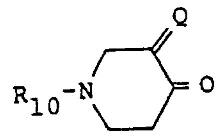

[XIX]

(wherein $R_{10}$ and Q have the same meanings as defined above) to prepare a compound represented by the general formula [XX]:

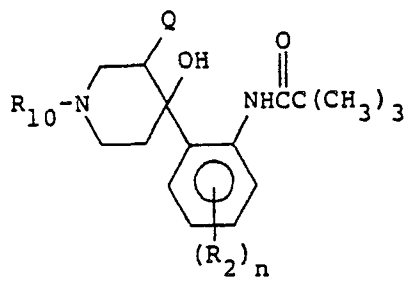

[XX]

(wherein $R_{10}$ $R_2$, n and Q have the same meanings as defined above). In the above reactions, the lithium compound formation is carried out at −5 to 30°C for 2 to 24 hours. An appropriate amount of the lithium compound-forming agent is 2 to 2.5 moles per mole of Compound [XVIII]. In the reaction with Compound [XIX], appropriate reaction temperature is −5 to 30°C, and appropriate reaction time is 2 to 24 hours. An appropriate amount of Compound [XIX] to be used is 1 to 1.5 moles per mole of Compound [XVIII]. Then, the resulting Compound [XX] is hydrolyzed in a mineral acid (hydrochloric acid, hydrobromic acid, hydroiodic acid, sulfuric acid, etc.) to obtain a compound represented by the general formula [XII]. The hydrolysis is carried out in 1 to 10N mineral acid, preferably in 2 to 6N mineral acid. Appropriate reaction temperature is between 50°C and the boiling point of the reaction solution, but the reaction is usually carried out at the boiling point of the reaction solution. The reaction is usually completed in 24 to 55 hours.

To complete the reaction, 72 hours to one week is appropriate as the reaction time.

Isolation and purification of the present compounds and starting compounds can be carried out according to the ordinary procedures used in the field of organic synthetic chemistry.

A pharmacologically acceptable acid addition salt of Compound [I] may be obtained by reacting Compound [I] with a suitable acid in a suitable solvent such as ethanol.

The pharmaceutical compositions of the present invention are described below.

It is obvious from the foregoing various experimental data that Compound [I′] has a hypotensive activity.

In view of the hypotensive activity, the compounds of the present invention may be used in various pharmaceutical forms for administration. Pharmaceutical compositions of the present invention are prepared by uniformly mixing an effective amount of the compound in the form of a base or an acid addition salt as an active ingredient with a pharmaceutically acceptable carrier. According to the pharmaceutical forms suitable for administration, the carrier may take various forms. It is desirable that the pharmaceutical compositions are in single administration form suitable for administration per os or by injection.

In preparation of the compositions for oral administration, any useful pharmaceutical carrier may be used. For example, water, glycols, oils or alcohols may be used to prepare oral liquid preparations such as suspensions and syrups, and excipients, lubricants, binders, disintegrators may be used to prepare powders, pills, capsules and tablets. Examples of the carriers are glucose and lactose as the excipients, starch and sodium alginate as the disintegrators, magnesium stearate, paraffin sulfate and talc as the lubricants, and syrup, ethanol and gelatin as the binders. The active ingredient is orally administered in a dose of 1—100 mg, particularly 10—60 mg, per day for an adult.

In the following Examples, which illustrate the various techniques hereinbefore described for the preparation of compounds according to this invention, the preparation is described of a number of compounds falling within the scope of the general formula I, but which are embraced by the provisos thereto and which are therefor excluded by them from the scope fo the present invention. Included in this category are the compounds whose preparation is described in Examples 1—3, 5, 7, 9, 10—13, 15, 18, 20 and 24. Neverthless the description of the preparation of those compounds is retained not least because many of those compounds are used as intermediates in the preparation of other compounds not covered by the provisos, and which therefore are included within the scope of the present invention. For the avoidance of doubt, in the following Examples the compounds embraced by the present invention are those the preparation of which is described in Examples 4, 6, 8, 14, 16, 17, 19 and 21—23. The procedures for the preparation of various starting compounds, and particularly the starting compounds used in Examples 10, 12 and 14, are described in Reference Examples 1—6.

Example 1

Production of 1′-benzoylmethyl-(2-oxo-1,2-dihydro-4H-3,1-benzoxazine-4-spiro-4′-piperidine):

In this example, 497.6 mq of ω-bromoacetophenone, 636.9 mg of 2-oxo-1,2-dihydro-4H-3,1-benzoxazine-4-spiro-4′-piperidine hydrochloride obtained according to the procedure described in Example 11, 12 ml of methanol and 0.7 ml of triethylamine were mixed together, and stirred at room temperature overnight. The white crystals deposited were separated by filtration, washed with 40 ml of water and dried to obtain 726.0 mg of the crude crystals of the desired compound. The crude crystals were recrystallized from chloroform to obtain 651.2 mg of the desired compound.

Example 2

Production of 1′-(2-phenyl-2-hydroxyethyl)-(2-oxo-1,2-dihydro-4H-3,1-benzoxazine-4-spiro-4′-piperidine):

In this example, 629 mg of 1′-benzoylmethyl-(2-oxo-1,2-dihydro-4H-3,1-benzoxazine-4-spiro-4′-piperidine) obtained according to the procedure described in Example 1 and 50 ml of ethanol were mixed together, and 300 mg of sodium borohydride is added thereto at room temperature with stirring. The resulting mixture was further stirred for 21 hours. Then, additional 100 mg of sodium borohydride was added thereto and the mixture was stirred for 15 hours. The white crystals deposited were separated by filtration, washed successively with 20 ml of methanol, with 30 ml of water, with 10 ml of methanol and with 10 ml of ether, and dried to obtain 518 mg of crystals. On the other hand, the mother liquor and the first methanol washings were combined together and concentrated under reduced pressure. The resulting crystalline residue was admixed with 10 ml of water, separated by filtration, washed with 20 ml of water, and dried to obtain 100 mg of crystals. These products were combined together and recrystallized from a mixed solvent of dimethylformamide and methanol (1:2 V/V) to obtain 445 mg of the desired compound.

Example 3

Production of 1′-(3,4-dimethoxybenzoylmethyl)-(2-oxo-1,2-dihydro-4H-3,1-benzoxazine-4-spiro-4′-piperidine):

In this example, 258.9 mg of 3,4-dimethoxy-ω-bromoacetophenone, 254.8 mg of 2-oxo-1,2-dihydro-4H-3,1-benzoxazine-4-spiro-4′-piperidine hydrochloride obtained according to the procedure described in Example 11, 5 ml of methanol, and 0.28 ml of triethylamine were mixed together, and stirred at room temperature overnight. The deposited crystals were separated by filtration, washed with 10 ml of water and dried to obtain 355 mg of crude crystals of the desired compound. The crystals were re-crystallized from a mixed solvent of chloroform and ethanol to obtain 315 mg of the desired compound.

Example 4

Production of 1′-[2-(3,4-dimethoxyphenyl)-2-hydroxyethyl]-(2-oxo-1,2-dihydro-4H-3,1-benzoxazine-4-spiro-4′-piperidine):

In this example, 1,090 mg of 1′-(3,4-dimethoxybenzoylmethyl)-(2-oxo-1,2-dihydro-4H-3,1-benzoxazine-4-spiro-4′-piperidine) obtained according to the procedure described in Example 3, 50 ml of ethanol, and 400 mg of sodium borohydride were mixed together, and stirred at room temperature overnight. Then, additional 100 mg of sodium borohydride was added thereto and the mixture was stirred at room temperature overnight. The white crystals deposited were separated by filtration, washed successively with 20 ml of methanol, with 30 ml of water, with 10 ml of methanol, and with 10 ml of ether, and dried to obtain 910 mg of crude crystals of the desired compound. On the other hand, the ethanol solution as the filtrate was concentrated under reduced pressure, and the resulting residue was admixed with 10 ml of water. The deposited crystals were separated by filtration, washed with 20 ml of water and dried to obtain 97 mg of crystals. These products were combined together and recrystallized from a mixed solvent of DMF and methanol to obtain 685 mg of the desired compound.

Example 5

Production of 1′-benzoylmethyl-(6-chloro-2-oxo-1,2-dihydro-4H-3,1-benzoxazine-4-spiro-4′-piperidine):

In this example, 398 mg of ω-bromoacetophenone, 578 mg of 6-chloro-2-oxo-1,2-dihydro-4H-3,1-benzoxazine-4-spiro-4′-piperidine hydrochloride obtained according to the procedure described in Example 13, 20 ml of ethanol and 0.56 ml of triethylamine were mixed together, and stirred at room temperature overnight. The reaction mixture was concentrated under reduced pressure, and the crystalline residue was triturated together with 10 ml of water and separated by filtration. The crystals were washed with 20 ml of water and dried to obtain 672 mg of crude crystals of the desired compound. The crude crystals were recrystallized from ethanol to obtain 368 mg of the desired compound.

Example 6

Production of 1′-(2-phenyl-2-hydroxyethyl)-(6-chloro-2-oxo-1,2-dihydro-4H-3,1-benzoxazine-4-spiro-4′-piperidine):

In this example, 572 mg of 1′-benzoylmethyl-(6-chloro-2-oxo-1,2-dihydro-4H-3,1-benzoxazine-4-spiro-4′-piperidine) obtained according to the procedure described in Example 5, 20 ml of ethanol and 572 mg of sodium borohydride were mixed together, and stirred at room temperature overnight. The deposited crystals were separated by filtration, and washed successively with 5 ml of ethanol and with 20 ml of water

to obtain 418 mg of crude crystals of the desired compound. On the other hand, the ethanol solution as the filtrate was concentrated under reduced pressure, and 50 ml of water was added to the residue. The deposited crystals were separated by filtration, washed with 20 ml of water and dried to obtain 95 mg of crystals. These products were combined together and recrystallized from a mixed solvent of DMF and ethanol to obtain 380 mg of the desired compound.

Example 7

Production of 1′-(4-chlorobenzoylmethyl)-(6-chloro-2-oxo-1,2-dihydro-4H-3,1-benzoxazine-4-spiro-4′-piperidine):

In this example, 467 mg of 4-chloro-ω-bromoacetophenone, 578 mg of 6-chloro-2-oxo-1,2-dihydro-4H-3,1-benzoxazine-4-spiro-4′-piperidine hydrochloride obtained according to the procedure described in Example 13, 10 ml of ethanol, and 0.56 ml of triethylamine were mixed together, and stirred at room temperature overnight. The deposited crystals were separated by filtration, washed successively with 5 ml of methanol and with 20 ml of water, and dried to obtain 692 mg of crude crystals of the desired compound. The crude crystals were recrystallized from a mixed solvent of DMF and ethanol to obtain 390 mg of the desired compound.

Example 8

Production of 1′-[2-(4-chlorophenyl)-2-hydroxyethyl]-(6-chloro-2-oxo-1,2-dihydro-4H-3,1-benzoxazine-4-spiro-4′-piperidine):

In this example, 650 mg of 1′-(4-chlorobenzoylmethyl)-(6-chloro-2-oxo-1,2-dihydro-4H-3,1-benzoxazine-4-spiro-4′-piperidine) obtained according to the procedure described in Example 7, and 20 ml of ethanol were mixed together, and 650 mg of sodium borohydride was added thereto at room temperature with stirring. The mixture was stirred at room temperature overnight. The desposited crystals were separated by filtration and washed successively with 5 ml ethanol and with 20 ml of water to obtain 517 mg of crude crystals of the desired compound. Then, 444 mg of the crude crystals were recrystallized from a mixed solvent of DMF and ethanol to obtain 397 mg of the desired compound.

Example 9

Production of 1′-[2-(4-chlorophenyl)-2-hydroxyethyl]-(2-oxo-1,2-dihydro-4H-3,1-benzoxazine-4-spiro-4′-piperidine]:

In this example, 700 mg of 4-chloro-ω-bromoacetophenone, 764 mg of 2-oxo-1,2-dihydro-4H-3,1-benzoxazine-4-spiro-4′-piperidine hydrochloride obtained according to the procedure described in Example 11, 30 ml of ethanol and 0.84 ml of triethylamine were mixed together, and stirred at room temperature overnight. The mixture was concentrated under reduced pressure, and 20 ml of water was added to the residue whereby the residue was solidified. The solid was separated by filtration, washed with 40 ml of water and admixed with 5 ml of methanol to crystallize it. The crystals were separated by filtration, washed with 5 ml of methanol and dried to obtain 425 mg of 1′-(4-chlorobenzoylmethyl)-(2-oxo-1,2-dihydro-4H-3,1-benzoxazine-4-spiro-4′-piperidine).

On the other hand, the filtrate (methanol layer) was concentrated under reduced pressure, and the resulting oily residue was admixed with 5 ml of ethyl acetate and again concentrated under reduced pressure. The resulting crystalline residue was triturated with 2 ml of ethyl acetate, separated by filtration, washed with 3 ml of ethyl acetate and dried to obtain 498 mg of crystals. Then, 800 mg of the crystals was used in the successive reduction reaction.

In the reduction, 800 mg of 1′-(4-chlorobenzoylmethyl)-(2-oxo-1,2-dihydro-4H-3,1-benzoxazine-4-spiro-4′-piperidine), 80 ml of ethanol and 800 mg of sodium borohydride were mixed together, and stirred at room temperature overnight. The resulting reaction mixture was concentrated under reduced pressure, and the residue was admixed with 10 ml of methanol and again concentrated under reduced pressure. The crystalline residue was triturated with 30 ml of water, and separated by filtration. The crystals were washed with 20 ml of water and dried to obtain 585 mg of crude crystals of the desired compound. The crude crystals were recrystallized from a mixed solvent of DMF and ethanol to obtain 383 mg of the desired compound.

Example 10

Production of 1′-benzyl-(2-oxo-1,2-dihydro-4H-3,1-benzoxazine-4-spiro-4′-piperidine):

In this example, 3.95 g of 1-benzyl-4-hydroxy-4-(2-aminophenyl) piperidine synthesized according to the procedure of Reference Example 1 and 40 ml of acetonitrile were mixed together, and 4.54 g of 1,1′-carbonyldiimidazole was added thereto with stirring at room temperature. The mixture was stirred at room temperature for 3 hours, additional 1 g of 1,1′-carbonyldiimidazole was added thereto, and the mixture was stirred for 2 hours. The deposited crude crystals of the desired compound were separated by filtration and washed with 10 ml of acetonitrile to obtain 2.90 g of crude crystals of the desired compound. On the other hand, the filtrate was concentrated under reduced pressure, and the residue was triturated together with water (20 ml × 2), and then admixed with 10 ml of methanol to crystallize it. The crystals were separated by filtration to obtain 0.38 g of crude crystals of the desired compound. Both crystals were combined together and recrystallized from ethanol to obtain 2.62 g of the desired compound.

Example 11

Production of 2-oxo-1,2-dihydro-4H-3,1-benzoxazine-4-spiro-4'-piperidine hydrochloride:

In this example, 736 mg of 1'-benzyl-(2-oxo-1,2-dihydro-4H-3,1-benzoxazine-4-spiro-4'-piperidine) synthesized according to the procedure of Example 10, 190 mg of 10% palladium-carbon, 7.14 ml of water, 2.38 ml of 1 N hydrochloric acid and 14.28 ml of methanol were mixed together, and hydrogen gas was bubbled into the mixture with stirring at 40°C for 4 hours and 30 minutes. Then, the reaction mixture was filtered to remove the palladium-carbon, and the filtrate was concentrated under reduced pressure to obtain 476 mg of a crystalline residue. The residue was recrystallized from methanol to obtain 188 mg of the desired compound.

Example 12

Production of 1'-benzyl-(6-chloro-2-oxo-1,2-dihydro-4H-3,1-benzoxazine-4-spiro-4'-piperidine):

In this example, 3.17 g of 1-benzyl-4-hydroxy-4-(2-amino-5-chlorophenyl) piperidine synthesized according to the procedure of Reference Example 2 and 30 ml of acetonitrile were mixed together, and 8.11 g of 1,1'-carbonyldiimidazole was added to the mixture with stirring at room temperature. The resulting mixture was stirred overnight. Then, the deposited crystals were separated by filtration and washed with 50 ml of water to obtain 2.5 g of crude crystals of the desired compound. The crude crystals were recrystallized from a mixed sovlent of dimethylformamide and methanol to obtain 1.8 g of the desired compound.

Example 13

Production of 6-chloro-2-oxo-1,2-dihydro-4H-3,1-benzoxazine-4-spiro-4'-piperidine:

In this example, 5 g of 1'-benzyl-(6-chloro-2-oxo-1,2-dihydro-4H-3,1-benzoxazine-4-spiro-4'-piperidine) synthesized according to the procedure described in Example 12, and 50 ml of 1,2-dichloroethane were mixed together, and 22.5 ml of a 1 M vinyl chlorocarbonate-ether solution was added to the mixture with stirring at room temperature. The resulting mixture was further stirred at rolm temperature for one hour, and then refluxed with heating for 4 hours. Then, additional 22.5 ml of the 1 M vinyl chlorocarbonate-ether solution was added thereto and the mixture was refluxed for additional one hour. The reaction mixture was cooled to room temperature, the deposited hydrochloride of the starting compound was removed by filtration, and the filtrate was washed with 30 ml of 1,2-dichloroethane. The filtrate was concentrated under reduced pressure, and the crystalline residue was triturated together with n-hexane (10 ml × 4) and then separated by filtration. The crystals were washed with 10 ml of n-hexane and dried to obtain 3.75 g of 1'-vinyloxycarbonyl-(6-chloro-2-oxo-1,2-dihydro-4H-3,1-benzoxazine-4-spiro-4'-piperidine).

Then, 1.84 g of the compound was mixed with 40 ml of methylene chloride. The resulting solution was admixed with 20 ml of a 5.71 N hydrogen chloride-ethyl acetate solution and allowed to stand at room temperature for one hour. The solution was concentrated under reduced pressure, the residue was admixed with 40 ml of ethanol, and the mixture was stirred at 50°C for one hour. The mixture was concentrated under reduced pressure. The resulting crystalline residue was subjected to trituration and decantation using respectively 40 ml of ether and 10 ml of ethanol. Then, 10 ml of ethanol was added to the resulting residue and the mixture was filtered. The crystals thus obtained were washed with 10 ml of ethanol and dried to obtain 1.0 g of crude crystals of the desired compound. The crude crystals were crystallized from ethanol to obtain 0.49 g of the desired compound.

Example 14

Production of 1'-methyl-(6-chloro-2-oxo-1,2-dihydro-4H-3,1-benzoxazine-4-spiro-4'-piperidine):

In this example, 1.69 g of 1-methyl-4-hydroxy-4-(2-amino-5-chlorophenyl) piperidine synthesized according to the procedure of Reference Example 3 was treated in the same manner as in Example 10, whereby 1.25 g of crude hydrochloride of the desired compound was obtained. The hydrochloride was recrystallized from methanol to obtain 0.57 g of hydrochloride of the desired compound.

Example 15

Production of 1'-[1-(3,4-dimethoxybenzoyl)ethyl]-(2-oxo-1,2-dihydro-4H-3,1-benzoxazine-4-spiro-4'-piperidine):

In this example, 2.73 g of 3,4-dimethoxy-α-bromo-propiophenone, 2.55 g of 2-oxo-1,2-dihydro-4H-3,1-benzoxazine-4-spiro-4'-piperidine hydrochloride synthesized according to the procedure of Example 11, 1.50 g of sodium iodide, 50 ml of ethanol and 6.16 ml of triethylamine were mixed together and stirred at room temperature for 6 days. The deposited crystals were separated by filtration, washed with ethanol and with water, and dried to obtain 2.77 g of the desired compound. For analytical purpose, 200 mg of the crystals were recrystallized from ethanol to obtain 94 mg of the pure product.

Example 16

Production of threo-1'-[2-(3,4-dimethoxyphenyl)-2-hydroxy-1-methylethyl]-(2-oxo-1,2-dihydro-4H-3,1-benzoxazine-4-spiro-4'-piperidine):

In this example, 1.23 g of 1'-[1-(3,4-dimethoxybenzoyl)ethyl]-(2-oxo-1,2-dihydro-4H-3,1-benzoxazine-4-spiro-4'-piperidine) and 60 ml of anhydrous tetrahydrofuran were mixed together, and 9 ml of 1M L-

selectride-tetrahydrofuran solution was added to the mixture with stirring at room temperature. The mixture were stirred at room temperature for one hour. Then, 2 ml of water was added to the reaction mixture and the mixture was concentrated under reduced pressure. The residue was admixed with 25 ml of water. The deposited crystals were separated by filtration and recrystallized from ethanol to obtain 1.10 g of the desired compound (yield: 88.8%).

Example 17

Production of erythro-1′-[2-(3,4-dimethoxyphenyl)-2-hydroxy-1-methylethyl]-(2-oxo-1,2-dihydro-4H-3,1-benzoxazine-4-spiro-4′-piperidine):

In this example, 1.23 g of 1′-[1-(3,4-dimethoxybenzoyl)ethyl]-(2-oxo-1,2-dihydro-4H-3,1-benzoxazine-4-spiro-4′-piperidine), 3 ml of 1N hydrochloric acid, 7.5 ml of water, 15 ml of methanol and 200 mg of 10% palladium-carbon were mixed together, and hydrogen gas was introduced to the mixture with stirring at 40°C for 26 hours. Then, the catalyst was filtered out and the filtrate was concentrated under reduced pressure. The crystalline residue was dissolved in a small amount of methanol, and the solution was rendered alkaline with aqueous sodium hydroxide solution and extracted with chloroform. The extract was concentrated under reduced pressure to obtain 820 mg of crystals of the desired compound. The crystals were twice recrystallized from ethanol to obtain 620 mg of the pure product.

Example 18

Production of 1′-[1-(3,4,5-trimethoxybenzoyl)-ethyl]-(2-oxo-1,2-dihydro-4H-3,1-benzoxazine-4-spiro-4′-piperidine):

In this example, the desired compound was obtained from 3,4,5-trimethoxy-α-bromopropiophenone and 2-oxo-1,2-dihydro-4H-3,1-benzoxazine-4-spiro-4′-piperidine hydrochloride according to the similar manner as described in Example 15.

Example 19

Production of threo-1′-[2-3,4,5-trimethoxyphenyl)-2-hydroxy-1-methylethyl]-(2-oxo-1,2-dihydro-4H-3,1-benzoxazine-4-spiro-4′-piperidine):

In this example, 1.32 g of 1′-[1-3,4,5-trimethoxybenzoyl)ethyl]-(2-oxo-1,2-dihydro-4H-3,1-benzoxazine-4-spiro-4′-piperidine) was treated in the similar manner as described in Example 16 to obtain 975 mg of the desired compound (yield: 73.5%).

Example 20

Production of 1′-[1-(3,4,6-trimethoxybenzoyl)ethyl]-(2-oxo-1,2-dihydro-4H-3,1-benzoxazine-4-spiro-4′-piperidine):

In this example, the desired compound was obtained from 3,4,6-trimethoxy-α-bromopropiophenone and 2-oxo-1,2-dihydro-4H-3,1-benzoxazine-4-spiro-4′-piperidine hydrochloride according to the similar manner as described in Example 15.

Example 21

Production of threo-1′-[2-(3,4,6-trimethoxyphenyl)-2-hydroxy-1-methylethyl]-(2-oxo-1,2-dihydro-4H-3,1-benzoxazine-4-spiro-4′-piperidine):

In this example, 1.32 g of 1′-[1-(3,4,6-trimethoxy-benzoyl)ethyl]-(2-oxo-1,2-dihydro-4H-3,1-benzoxazine-4-spiro-4′-piperidine) was treated in the similar manner as described in Example 16 to obtain 838 mg of the desired compound (yield: 63.2%).

Example 22

Production of erythro-1′-[2-(3,4,6-trimethoxyphenyl)-2-hydroxy-1-methylethyl]-(2-oxo-1,2-dihydro-4H-3,1-benzoxazine-4-spiro-4′-piperidine):

In this example, 1.1 g of 1′-[1-(3,4,6-trimethoxybenzoyl)ethyl]-(2-oxo-1,2-dihydro-4H-3,1-benzoxazine-4-spiro-4′-piperidine) and 30 ml of ethanol were mixed together, and 945.8 mg of sodium borohydride was added to the mixture with stirring at room temperature. The mixture was stirred at room temperature overnight and concentrated under reduced pressure. Water was added to the mixture and the mixture was extracted with chloroform. The extract was concentrated under reduced pressure and the residue was subjected to silica gel column chromatography (Wako Gel C-200, ethyl acetate) to obtain 257 mg of the desired compound and 661 mg of the threo isomer. The desired compound was recrystallized from ethanol to obtain 200 mg of the pure product.

Example 23

Production of 1′-[2-(3,4,5-trimethoxyphenyl)-2-hydroxyethyl]-(2-oxo-1,2-dihydro-4H-3,1-benzoxazine-4-spiro-4′-piperidine):

In this example, 867 mg of 3,4,5-trimethoxy-ω-bromoacetophenone, 764 mg of 2-oxo-1,2-dihydro-4H-3,1-benzoxazine-4-spiro-4′-piperidine hydrochloride, 0.84 ml of triethylamine and 20 ml of ethanol were mixed together, and stirred at room temperature overnight. Then, 1 g of sodium borohydride was added to the reaction mixture and the mixture was further stirred at room temperature overnight. The deposited

crystals were separated by filtration, and washed successibly with methanol and with water to obtain 482 mg of crude crystals of the desired compound. On the other hand, the filtrate was concentrated under reduced pressure and the residue was dissolved in chloroform. The solution was washed with water and concentrated under reduced pressure. The resultant oily residue was subjected to silica gel chromatography (Wako Gel C-200, ethyl acetate) to obtain 93 mg of crude crystals of the desired compound. These crude crystals were combined together and recrystallized from ethanol to obtain 465 mg of the desired compound.

Example 24

Production of 1′-[2-(3-hydroxy-4-methoxyphenyl)-2-hydroxyethyl]-(2-oxo-1,2-dihydro-4H-3,1-benzoxazine-4-spiro-4′-piperidine):

In this example, 1.82 g of 3-acetoxy-4-methoxy-ω-bromoacetophenone, 1.53 g of 2-oxo-1,2-dihydro-4H-3,1-benzoxazine hydrochloride, 1.68 ml of triethylamine and 30 ml of ethanol were mixed together, and stirred at room temperature overnight. The reaction mixture was concentrated under reduced pressure and the admixed with water. The deposited crystals were separated by filtration and dried to obtain 1.78 g of crude crystals of 1′-(3-hydroxy-4-methoxybenzoylmethyl)-(2-oxo-1,2-dihydro-4H-3,1-benzoxazine-4-spiro-4′-piperidine). Then, 1 g of crude crystals were mixed with 50 ml of ethanol, 1.0 g of sodium borohydride was added to the mixture with stirring at room temperature, and the mixture was stirred at room temperature overnight. The reaction mixture was concentrated under reduced pressure and water was added to the residue. The deposited crystals of the desired compound were separated by filtration and recrystallized from dimethylformamide-ethanol to obtain 307 mg of the pure product.

Example 25

(Example of preparing 10,000 5 mg-tablets)

| | |
|---|---|
| Compound 6 | 50 g |
| Magnesium stearate | 4 g |
| Crystalline cellulose | 746 g |

The above-described ingredients are mixed for 5 minutes by means of a mixer. The resulting mixed powder is made into 10,000 tablets of 6.0 mm in diameter, 2.5 mm in thickness, and 80 mg in weight using a tablet-making machine (Model HU-37; made by Kikusui Seisakusho) equipped with a pestle having a plane surface and round corners.

Example 26

(Example of preparing a powder)

| | |
|---|---|
| Compound 8 | 110 g |
| Lactose | 890 g |

The above-described ingredients are mixed for 10 minutes using a mixer to obtain a uniform mixture (powder).

Reference Example 1

Production of 1-benzyl-4-hydroxy-4-(2-aminophenyl) piperidine:

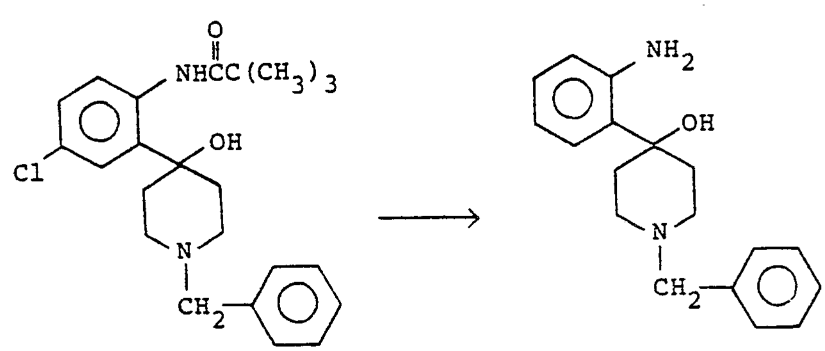

In this reference example, 50 g of 1-benzyl-4-hydroxy-4-(2-pivaloylamino-5-chlorophenyl) piperidine, 2.5 g of 10% palladium-carbon, 10 g of sodium hydroxide and 1 l of ethanol were mixed together, and hydrogen gas was bubbled into the mixture with stirring at room temperature for 10 hours and 30 minutes.

Then, additional 2.5 g of 10% palladium-carbon was added to the mixture, and hydrogen gas was bubbled thereinto for additional 19 hours. After it was confirmed by silica gel thin layer chromatography that the starting compound disappeared, the reaction was discontinued and the palladium-carbon was removed by filtration. The filtrate was concentrated under reduced pressure, the residue was admixed with 750 ml of 2 N sulfuric acid and the mixture was refluxed with heating for 3 days. The reaction mixture was adjusted to pH 10 with an aqueous concentrated sodium hydroxide solution, and the deposited insolubles were separated by filtration, washed with 100 ml of water and dissolved in 400 ml of chloroform. The solution was washed with water (100 ml × 4), dried and concentrated under reduced pressure. The resulting oily residue was admixed with 6 ml of ethyl acetate and the mixture was concentrated under reduced pressure. The residue was admixed with 30 ml of n-hexane to crystallize the desired compound. The crystals were separated by filtration, washed with 150 ml of n-hexane and dried to obtain 14.4 g of crude crystals of the desired compounds. The crude crystals were recrystallized from a mixed solvent of n-hexane and ethyl acetate (3:1 V/V) to obtain 10.4 g of the desired compound.

Melting point: 121.0—122.5°C

Elemental analysis:

| | H | C | N |
|---|---|---|---|
| Calculated: | 7.85% | 76.56% | 9.92% |
| Found: | 7.95% | 76.60% | 9.97% |

Reference Example 2

Production of 1-benzyl-4-hydroxy-4-(2-amino-5-chlorophenyl) piperidine:

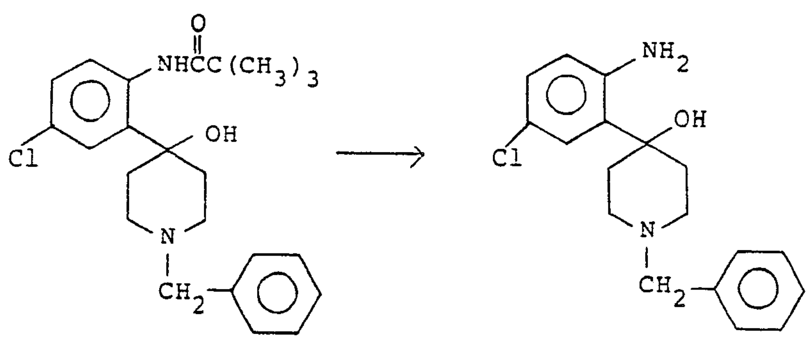

In this reference example, 10 g of 1-benzyl-4-hydroxy-4-(2-pivaloylamino-5-chlorophenyl) piperidine and 300 ml of 2 N sulfuric acid were mixed together, and refluxed with heating for 48 hours. The reaction mixture was poured into 500 ml of ice water, and the resulting mixture was adjusted to pH 10 with an aqueous concentrated sodium hydroxide solution. The deposited insolubles were separated by filtration, washed with 300 ml of water and dissolved in 200 ml of chloroform. The solution was washed with water (50 ml × 4), dried and concentrated under reduced pressure. The resulting oily residue (7.5 g) was admixed with 7.5 ml of ethyl acetate and 52.5 ml of n-hexane. The mixture was concentrated under reduced pressure. The crystallized residue was triturated together with 9 ml of a mixed solvent of ethyl acetate and n-hexane (1:5 V/V), and the mixture was filtered. The crystals were washed with the same mixed solvent and dried to obtain 2.19 g of crystals. Then, the crystals were recrystallized from a mixed solvent of ethyl acetate and n-hexane (1:3 V/V) to obtain 1.5 g of the desired compound.

Melting point: 100.0—101.0°C

Elemental analysis:

| | H | C | N |
|---|---|---|---|
| Calculated: | 6.68% | 68.24% | 8.84% |
| Found: | 6.96% | 68.41% | 8.57% |

Reference Example 3

Production of 1-methyl-4-hydroxy-4-(2-amino-5-chlorophenyl) piperidine:

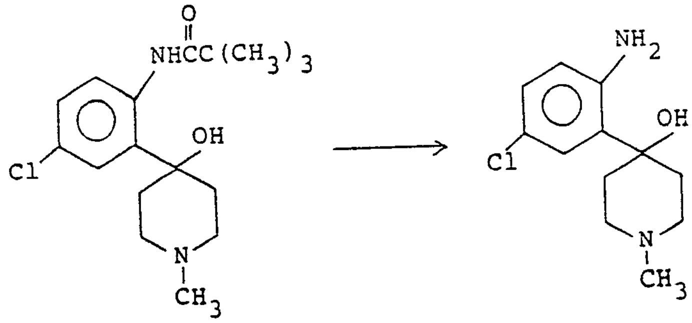

In this reference example, 9.9 g of 1-methyl-4-hydroxy-4-(2-pivaloylamino-5-chlorophenyl) piperidine and 150 ml of 2 N sulfuric acid were mixed together, and refluxed with heating for 54 hours. The reaction mixture was adjusted to pH 10 with an aqueous concentrated sodium hydroxide solution, and then extracted with ethyl acetate (100 ml × 4). The organic layer was washed with an aqueous saturated sodium chloride solution, and concentrated under reduced pressure. The resulting crystalline residue was admixed with 20 ml of n-hexane, triturated, separated by filtration, washed with 100 ml of n-hexane, and dried to obtain 4.9 g of crude crystals of the desired compound. The crystals were recrystallized from ethyl acetate to obtain 1.91 g of a pure product.

Melting point: 199.5—200.5°C

Elemental analysis:

| | C | H | N |
|---|---|---|---|
| Calculated: | 59.62% | 7.09% | 11.59% |
| Found: | 59.76% | 7.21% | 11.38% |

Reference Example 4

Production of 1-methyl-4-(2-amino-5-chlorophenyl)-1,2,5,6-tetrahydropyridine:

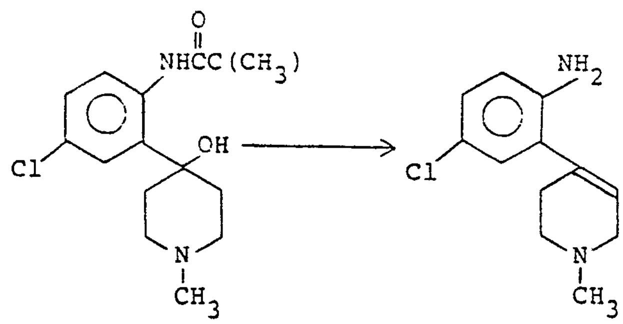

In this reference example, 30.7 g of 1-methyl-4-hydroxy-4-(2-pivaloylamino-5-chlorophenyl) piperidine and 300 ml of 2 N sulfuric acid were mixed together, and the mixture was refluxed with heating for 72 hours. The reaction mixture was adjusted to pH with an aqueous concentrated sodium hydroxide solution, and extracted with ethyl acetate (200 ml × 3). The extract was washed with an aqueous saturated sodium chloride solution (100 ml × 2), dried and concentrated under reduced pressure. The residue was admixed with 50 ml of ethyl acetate, and the deposited crystals of 1-methyl-4-hydroxy-(2-amino-5-chlorophenyl) piperidine was washed with 50 ml of petroleum ether to obtain 5.1 g of crystals.

Then, the filtrates were combined together and concentrated. The residue was dissolved in 25 ml of ethyl acetate, and an appropriate amount of n-hexane was added thereto. The deposited product was separated by filtration to obtain 2.3 g of 1-methyl-4-hydroxy-(2-amino-5-chlorophenyl) piperidine. The filtrate was concentrated under reduced pressure, the residue was dissolved in a small amount of chloroform, and the solution was charged onto a column of 6 cm in inner diameter packed with a suspension of 181 g of Wako gel C-200 [made by Wako Junyaku Co.) in chloroform. Elution was carried out first with 2 l of chloroform and then with 3 l of chloroform-methanol (20:1 V/V) to obtain a fraction containing the desired compound. The fraction was concentrated under reduced pressure, the residue was admixed with 25 ml of ethyl acetate and 25 ml of n-hexane, and the mixture was concentrated under reduced pressure. The concentrate was allowed to stand at room temperature for two hours, and the crystallized residue was admixed with 5 ml of n-hexane, triturated and separated by filtration. The crystals were washed with 25 ml of n-hexane to obtain 4.96 g of 1-methyl-4-(2-amino-5-chlorophenyl)-1,2,5,6-tetrahydropyridine. The crystals had a purity high enough to be used in the successive reaction, but 200 mg of the crystals was recrystallized from a mixed solvent of 0.2 ml of ethyl acetate and 0.5 ml of petroleum ether to obtain 63.5 mg of a pure product.

Melting point: 75.0—76.4°C

Elemental analysis:

| | H | C | N |
|---|---|---|---|
| Calculated: | 6.78% | 64.71% | 12.57% |
| Found: | 6.87% | 64.71% | 12.44% |

Reference Example 5

Production of 1-benzyl-4-hydroxy-4-(2-pivaloylamino-5-chlorophenyl) piperidine:

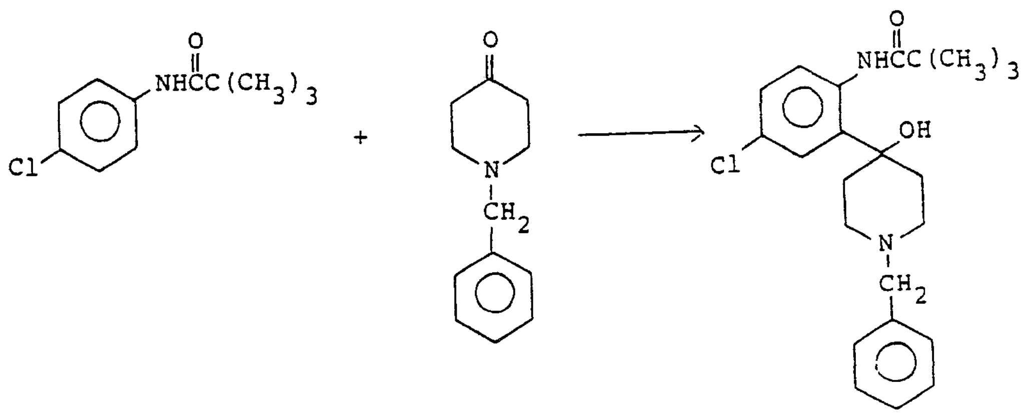

In this reference example, 31.8 g of 1-pivaloyl-amino-4-chlorobenzene and 750 ml of anhydrous tetrahydrofuran were mixed together, and 240 ml of 15% n-butyl lithium was added dropwise to the mixture over a period of 35 minutes with stirring a −5 to 0°C in a nitrogen gas stream. After the dropwise addition, the mixture was further stirred at 0°C for 2 hours, and the solution prepared by dissolving 30 g of 1-benzyl-4-piperidone in 45 ml of anhydrous tetrahydrofuran was added dropwise thereto at 0—3°C over a period of one hour. The mixture was stirred at 0°C for 3 hours. Then, the cooling was discontinued, and the mixture was stirred at room temperature overnight. The reaction mixture was poured into 1 l of ice water. The resulting organic layer was separated, washed with an aqueous saturated sodium chloride solution, dried and concentrated under reduced pressure. The residue was subjected to silica gel chromatography (ethyl acetate: n-hexane = 1:4, and then only ethyl acetate) to obtain 23.1 g of crude crystals of the desired compound. The crude crystals were recrystallized from a mixed solvent of ethyl acetate and n-hexane (14:25 V/V) to obtain 16.7 g of the desired compound.

Melting point: 202.0—202.7°C

Elemental analysis:

| | H | C | N |
|---|---|---|---|
| Calculated: | 7.29% | 68.90% | 6.99% |
| Found: | 7.38% | 68.85% | 7.08% |

Reference Example 6

Production of 1-methyl-4-hydroxy-4-(2-pivaloylamino-5-chlorophenyl) piperidine:

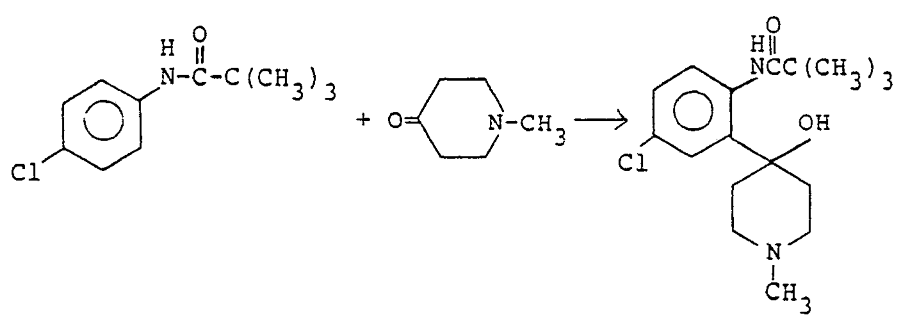

In this reference example, 2.12 g of 1-pivaloylamino-4-chlorobenzene and 60 ml of anhydrous tetrahydrofuran were mixed together, and 16 ml of 15% n-butyllithium was added dropwise to the mixture over a period of 15 minutes with stirring at −5 to 0°C in a nitrogen gas stream. After the dropwise addition, the mixture was further stirred at 0°C for 2 hours, and the solution prepared by dissolving 1.13 g of 1-methyl-4-piperidone in 3 ml of anhydrous tetrahydrofuran was added dropwise thereto over a period of one hour at about 0°C. Then, the mixture was further stirred at 0°C for 2 hours, and then the cooling was discontinued. The mixture was stirred at room temperature overnight. The reaction mixture was poured into 200 ml of ice water, and the mixture was made acidic with concentrated hydrochloric acid and extracted with 100 ml of ethyl acetate. The organic layer was further extracted with 60 ml of 1 N hydrochloric acid. All the aqueous layers were combined together, made alkaline with a concentrated sodium hydroxide solution, and then extracted with 100 ml of ethyl acetate. The organic layer was washed with water and concentrated under reduced pressure to obtain 1.3 g of crude-crystals of the desired compound. The crude crystals were recrystallized from methanol to obtain 0.52 g of a pure product.

**Claims**

1. Piperidine derivatives of the formula [I]:

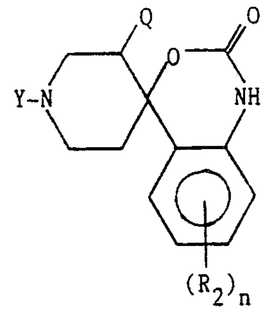

[I]

wherein Y is a $C_1$—$C_3$ alkyl or a group represented by

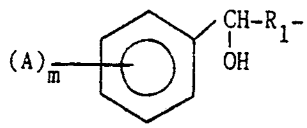

wherein m is 0 or an integer of 1—5; A is hydroxyl, halogen, $C_1$—$C_5$ alkyl, $C_1$—$C_5$ alkoxy, $C_2$—$C_5$ alkenyloxy, $C_2$—$C_5$ alkynyloxy, $C_1$—$C_5$ alkylthio, carboxyl, $C_1$—$C_5$ alkoxycarbonyl, nitro, amino, $C_1$—$C_5$ alkylamino, $C_1$—$C_5$ alkanoyl-amino, sulfamoyl, mono or di-($C_1$—$C_5$ alkyl) amino-sulfonyl, $C_1$—$C_5$ alkylsulfonyl, carbamoyl, cyano or trifluoromethyl; when m is 2 or more, the A groups may be the same or different, or two A groups may be combined to form a $C_1$—$C_5$ alkylenedioxy group; $R_1$ is straight-chain alkylene having 1—4 carbon atoms, or straight-chain alkylene having 1—4 carbon atoms and having a $C_1$—$C_5$ alkyl substituent; Q is hydrogen, $C_1$—$C_5$ alkyl, halogen, hydroxyl or $C_1$—$C_5$ alkanoyloxy; n is 0 or an integer of 1—4; $R_2$ is hydroxyl, $C_1$—$C_5$ alkoxy, halogen, trifluoromethyl, trifluoromethoxy, trifluoromethylthio, nitro or amino; when n is 2 or more, the $R_2$ groups may be the same or different, or two $R_2$ groups may be combined to form a $C_1$—$C_5$ alkylenedioxy; with the proviso that when Y is said

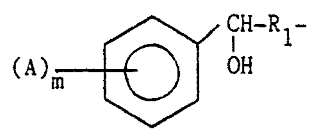

group,
then Q is H and, if none of the $R_2$ groups is halogen, at least two of the A groups in

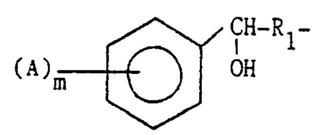

must be alkoxy; and acid addition salts thereof.

2. A compound according to claim 1, represented by the general formula (I''):

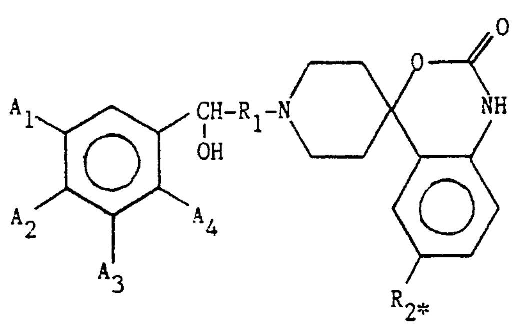

[I'']

wherein $A_1$, $A_2$, $A_3$ and $A_4$ are the same or different groups and are hydrogen or a group as defined for A; $R_2$* is hydrogen or a group as defined for $R_2$; and $R_1$ is as defined in claim 1, it being provided that either $R_2$

is halogen or at least two of $A_1$—$A_4$ are $C_1$—$C_5$ alkoxy, or a pharmaceutically acceptable addition salt thereof.

3. A compound or salt according to claim 2 wherein $A_1$, $A_2$, $A_3$ and $A_4$ are hydrogen, or at least one of $A_1$, $A_2$, $A_3$ and $A_4$ is $C_1$—$C_5$ alkoxy or halogen.

4. A compound or salt according to claim 2 or 3, wherein $R_1$ is methylene or methylmethylene.

5. A pharmaceutical composition comprising a pharmaceutically acceptable carrier and a compound of the formula

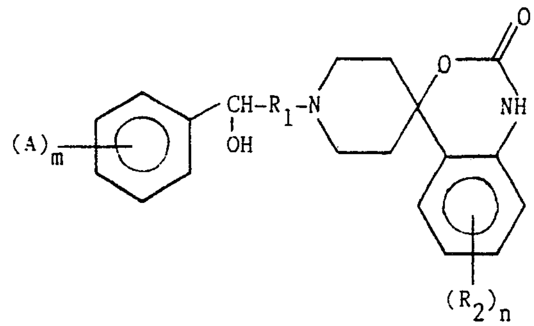

[I']

where A, $R_1$, $R_2$, m and n are as defined in claim 1, or a pharmaceutically acceptable acid addition salt thereof.

**Patentansprüche**

1. Piperidin-Derivate der Formel (I)

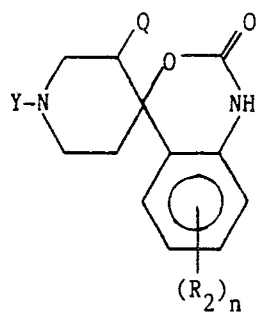

[I]

in der Y einen $C_1$—$C_3$-Alkylrest oder einen Rest der Formel

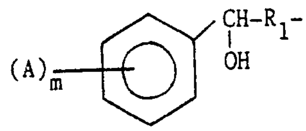

bedeutet, in der m den Wert 0 hat oder eine ganze Zahl im Wert von 1 bis 5 ist; A eine Hydroxylgruppe, ein Halogenatom, einen $C_1$—$C_5$-Alkyl-, $C_1$—$C_5$-Alkoxy-, $C_2$—$C_5$-Alkenyloxy, $C_2$—$C_5$-Alkinyloxy-, $C_1$—$C_5$-Alkylthio-, Carboxyl-, $C_1$—$C_5$-Alkoxycarbonyl-, Nitro, Amino-, $C_1$—$C_5$-Alkylamino-, $C_1$—$C_5$-Alkanoylamino-, Sulfamoyl-, Mono- oder Di-($C_1$—$C_5$-alkyl)-aminosulfonyl-, $C_1$—$C_5$-Alkylsulfonyl-, Carbamoyl-, Cyan- oder Trifluormethylrest darstellt; wenn m einen Wert von 2 oder mehr hat, die Reste A gleich oder verschieden sein können oder zwei Reste A zu einem $C_1$—$C_5$-Alkylendioxyrest vereinigt sein können; $R_1$ einen unverzweigten Alkylenrest mit 1 bis 4 Kohlenstoffatomen oder einen unverzweigten Alkylenrest mit 1 bis 4 Kohlenstoffatomen und einem $C_1$—$C_5$-Alkyl-substituenten bedeutet, Q ein Wasserstoffatom, einen $C_1$—$C_5$-Alkylrest, ein Halogenatom, eine Hydroxylgruppe oder einen $C_1$—$C_5$-Alkanoyloxyrest darstellt; n den Wert 0 hat oder eine ganze Zahl im Wert von 1 bis 4 ist; $R_2$ eine Hydroxyl-, $C_1$—$C_5$-Alkoxy-, Halogen-, Trifluormethyl-, Trifluormethoxy-, Trifluormethylthio-, Nitro- oder Aminogruppe bedeutet; wenn n den Wert 2 oder mehr hat, die Reste $R_2$ gleich oder verschieden sein können oder zwei Reste $R_2$ zu einem $C_1$—$C_5$-Alkylendioxyrest verbunden sein können; mit der Maßgabe, daß Q ein Wasserstoffatom bedeutet, wenn Y den Rest

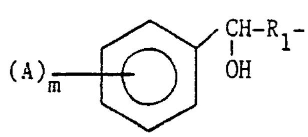

bedeutet, und daß mindestens zwei Reste A in der Formel

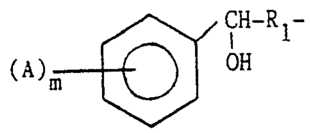

Alkoxyreste bedeuten müssen, wenn keiner der Reste $R_2$ ein Halogenatom darstellt; und die Säureadditionssalze davon.

2. Verbindung nach Anspruch 1 der allgemeinen Formel (I'')

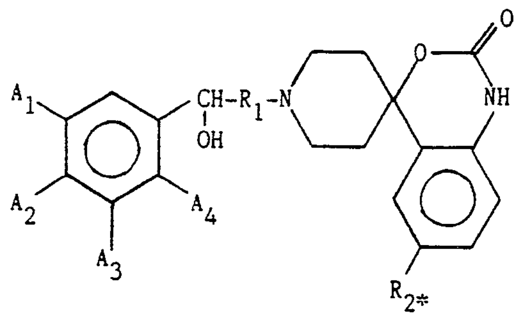

[I'']

in der $A_1$, $A_2$, $A_3$ und $A_4$ gleich oder verschieden sind und Wasserstoffatome oder einen Rest mit der Definition von A darstellen; $R_2$* ein Wasserstoffatom oder einen Rest mit der Definition von $R_2$ bedeutet; und $R_1$ wie in Anspruch 1 definiert ist, mit der Maßgabe, daß entweder $R_2$ ein Halogenatom bedeutet oder mindestens zwei der Reste $A_1$ bis $A_4$ $C_1$—$C_5$-Alkoxyreste bedeuten; oder pharmazeutisch verträgliche Säureadditionssalze davon.

3. Eine Verbindung oder ein Salz nach Anspruch 2, in der $A_1$, $A_2$, $A_3$ und $A_4$ Wasserstoffatome bedeuten oder mindestens einer der Reste $A_1$, $A_2$, $A_3$ und $A_4$ einen $C_1$—$C_5$-Alkoxyrest oder ein Halogenatom darstellen.

4. Eine Verbindung oder ein Salz nach Anspruch 2 oder 3, in der $R_1$ eine Methylen- oder Methylmethylengruppe bedeutet.

5. Arzneimittel, umfassend einen pharmazeutisch verträglichen Träger und eine Verbindung der Formel (I')

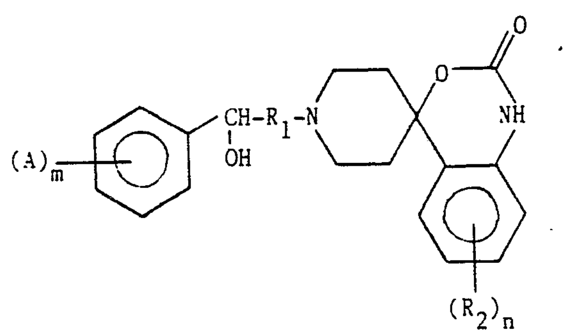

[I']

in der A, $R_1$, $R_2$, m und n wie in Anspruch 1 definiert sind, oder ein pharmazeutisch verträgliches Säureadditionssalz davon.

**Revendications**

1. Dérivé de pipéridine de formule (I):

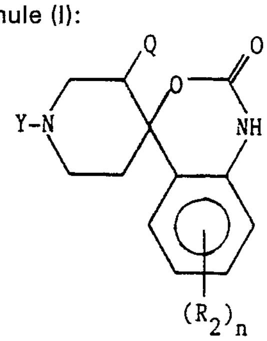

[I]

dans laquelle Y est un groupe alkyle en $C_{1-3}$ ou un groupe représenté par

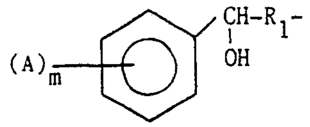

dans lequel m est 0 ou un nombre entier de 1 à 5; A est un groupe hydroxyle, halogéno, alkyle en $C_{1-5}$, alcoxy en $C_{1-5}$, alcényloxy en $C_{2-5}$, alcynyloxy en $C_{2-5}$, alkylthio en $C_{1-5}$, carboxyle, alcoxycarbonyle en $C_{1-5}$, nitro, amino, alkylamino en $C_{1-5}$, alcanoylamino en $C_{1-5}$, sulfamoyle, mono- ou di-(alkyl en $C_{1-5}$)aminosulfonyle, alkylsulfonyle en $C_{1-5}$, carbamoyle, cyano ou trifluorométhyle; lorsque m est égal ou supérieur à 2, les groupes A peuvent être identiques ou différents, ou deux groupes A peuvent être pris conjointement pour former un groupe alkylène-dioxy en $C_{1-5}$; $R_1$ est un groupe alkylène à chaîne droite comportant 1 à 4 atomes de carbone, ou un groupe alkylène à chaîne droite comportant 1 à 4 atomes de carbone et ayant un substituent alkyle en $C_{1-5}$; Q est un hydrogène, un groupe alkyle en $C_{1-5}$, halogéno, hydroxyle ou alcanoyloxy en $C_{1-5}$; n est 0 ou un nombre entier de 1 à 4; $R_2$ est un groupe hydroxyle, alcoxy en $C_{1-5}$, halogèno, trifluorométhyle, trifluorométhoxy, trifluorométhylthio, nitro ou amino; lorsque n est supérieur ou égal à 2, les groupes $R_2$ peuvent être identiques ou différents, ou deux groupes $R_2$ peuvent être pris conjointement pour former un groupe alkylène-dioxy en $C_{1-5}$; à condition que, lorsque Y est le dit groupe

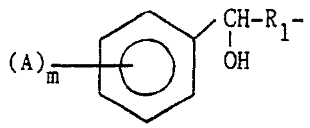

Q soit alors H et que, si aucun des groupes $R_2$ n'est un halogène, au moins deux des groupes A dans

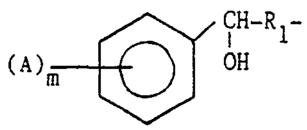

soient des groupes alcoxy; et leurs sels d'addition avec un acide.

2. Composé selon la revendication 1, représenté par la formule générale (I''):

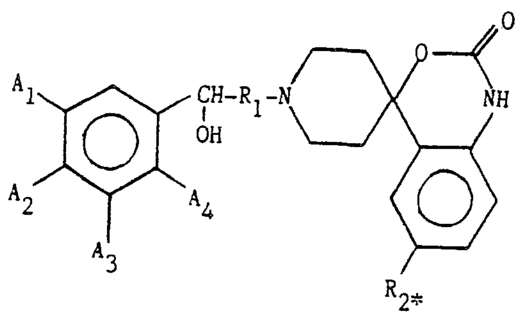

[I'']

dans laquelle $A_1$, $A_2$, $A_3$ et $A_4$ sont des groupes identiques ou différents et sont chacun un hydrogène ou un groupe tel que défini pour A; $R_2$* est un hydrogène ou un groupe tel que défini pour $R_2$; et $R_1$ est tel que défini dans la revendication 1, à condition que $R_2$ soit un halogène ou qu'au moins deux des groupes $A_1$—$A_4$ soient des groupes alcoxy en $C_{1-5}$, ou un de ses sels d'addition pharmaceutiquement acceptable.

3. Composé ou sel selon la revendication 2, dans lequel $A_1$, $A_2$, $A_3$ et $A_4$ sont chacun un hydrogène, ou au moins un des groupes $A_1$, $A_2$, $A_3$ et $A_4$ est un groupe alcoxy en $C_{1-5}$ ou un halogène.

4. Composé ou sel selon la revendication 2 ou 3, dans lequel $R_1$ est un groupe méthylène ou méthylméthylène.

5. Composition pharmaceutique comprenant un véhicule pharmaceutiquement acceptable et un composé de formule

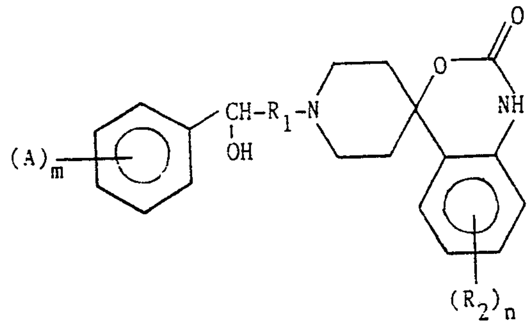

[I']

dans laquelle A, $R_1$, $R_2$, m et n sont tels que définis dans la revendication 1, ou un de ses sels d'addition avec un acide, pharmaceutiqement acceptable.